# EUROPEAN PATENT SPECIFICATION

(11) **EP 0 585 553 B1**
(45) Date of publication and mention of the grant of the patent: **15.09.1999**
(21) Application number: 93109790.1
(22) Date of filing: 18.06.1993
(51) Int. Cl.: A61N 1/05

(54) **Multipolar electrode lead**
Mehrpolige Elektrodenleitung
Fil d'électrode multipolaire

(30) Priority: 14.08.1992 SE 9202351; 30.09.1992 SE 9202824
(43) Date of publication of application: 09.03.1994
(73) Proprietor: Pacesetter AB, 171 95 Solna (SE)
(72) Inventor: Lindegren, Ulf, S-122 35 Enskede (SE); Freller, Helmut, W-8585 Röthenbach (DE); Lorenz, Hans-Peter, W-8501 Schwarzenbruck (DE)
(74) Representative: Lettström, Richard Wilhelm

(56) References cited:
- EP-A- 0 092 797
- CH-A- 656 313
- DE-A- 3 031 752
- US-A- 4 437 474
- US-A- 4 662 382
- US-A- 4 947 866
- PARAT Index of Polymer Trade Names, VCH-Berlin

## Description

This invention relates to a multipolar electrode lead for a medical apparatus devised to deliver stimulation pulses to living tissue, said electrode lead comprising an insulating sheath in which at least a first conductor and a second conductor are coiled, at least the first conductor being covered with an insulating coating to electrically separate the conductors from one another.

One such known electrode lead is described in DE-A-3 031 752. The known electrode lead contains a plurality of electrode conductors each of which provided with an insulating coating of a polymer or the like to electrically separate the respective conductors. The insulated conductors are coiled next to one another at the same distance from the electrode lead's center axis.

After implantation in a patient's vascular system, the known electrode lead is bent back and forth by movements of the patient's body and blood vessels. The insulating polymer coating could then rub off between two conductors, thereby leading to a short-circuit.

US-A-4 437 474 discusses the problem when winding insulated wires on a mandrel where the pressure exerted on the wire causes the wires to cut through the insulation in a cheese cutting manner. Till's is solved by the winding of each wire separately whithout any coating and by thereafter coating each coil with an insulating material.

The object of the invention is to provide an electrode lead, as described above, capable of withstanding high mechanical stress without any short-circuit developing between two conductors.

This is achieved in accordance with the invention in that the electrode lead, as described above, is devised so that the insulated coating on the first conductor is electrically insulating and abrasion-resistant.

The electrically insulating and abrasion-resistant coating insulates the conductors from one another while reducing the risk of short-circuiting. Since the electrically insulating and abrasion-resistance coating can be made thinner than an insulating polymer coating, which must be thicker than is electrically warranted in order to reduce the risk of short-circuiting, the entire electrode lead can be made thinner.

In one refinement of the electrode lead according to the invention, the second conductor consists of a separate conductor wire provided with the same electrically insulating and abrasion-resistant coating as the first conductor.

Since both conductors are provided with an electrically insulating and abrasion-resistant coating, electrical insulation between the conductors is further enhanced.

The electrically insulating and abrasion-resistant coating is preferably made from DLC (diamond like carbon), a material with electrically insulating and abrasion-resistant properties. In this context, it would be an advantage if the electrically insulating and abrasion-resistant coating were applied in thicknesses ranging from 0.1 to 10 µm, preferably between 1 and 5 µm.

Alternatively the electrically insulating and abrasion-resistant coating may, in accordance with the invention, be achieved in that the insulating coating comprises a first layer of an electrically insulating material and a second of an abrasion-resistant material.

As a result of the abrasion-resistant layer, the electrically insulating layer is not exposed to any abrasion stress when the electrode lead bends back and forth but is protected by the abrasion-resistant coating. Therefore, the electrically insulating layer may consist of a thin layer of a polymer.

When the two conductors are provided with an electrically insulating layer on which the abrasion-resistant layer is deposited, the diameter of the respective conductor can be reduced, as the electrically insulating layers can be devised and optimized on the basis of their electrical properties without regard to the mechanical wear taken over by the abrasion-resistant coating. Assuming that it is normal to use polymer coating thicknesses exceeding 60 µm, these coatings can, according to an embodiment of the invention, be reduced to about 10 µm to electrically insulate the conductors from one another.

The abrasion-resistant coating is preferably made from a material such as titanium nitride, aluminum oxide or DLC (diamond-like carbon) whose abrasion-resistant properties are known. In this context, it would be an advantage if the abrasion-resistant coating is deposited in a thickness of from 0.1 to 10 µm, preferably from 1 to 5 µm.

The invention will be described in greater detail below with reference to four figures, whereby:
FIG. 1 shows a first embodiment of the electrode lead according to the invention,
FIG. 2 shows an enlargement of a cross-section of two electrode conductors in the electrode lead according to FIG. 1,
FIG. 3 shows a second embodiment of the electrode lead according to the invention, and
FIG. 4 shows an enlargement of a cross-section of two electrode conductors in the electrode lead according to FIG. 3.

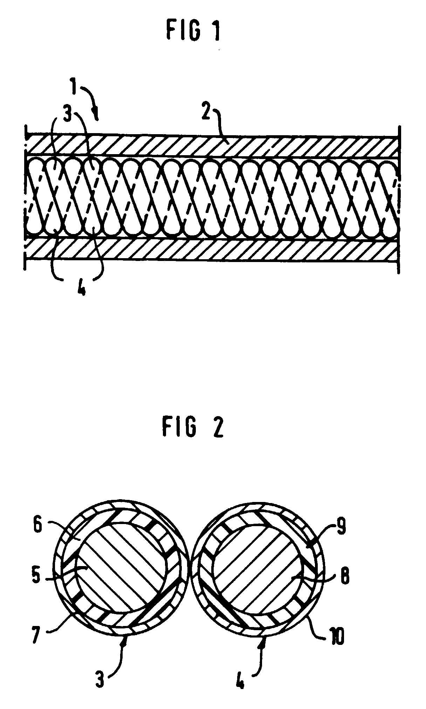

FIG. 1 shows an electrode lead 1 which consists of an insulating sheath 2, a first conductor 3 and a second conductor 4. The figure only shows a midsection of the electrode lead 1 which has a connection contact at one end for connecting the electrode lead 1 to a stimulation pulse generator (not shown), such as a pacemaker, and at least one electrode at the other end for e.g. delivering stimulation pulses from the stimulation pulse generator to surrounding tissue, such as a heart.

The first conductor 3 and the second conductor 4 have the cross-sections shown in FIG. 2. The first conductor 3 comprises a conductor wire 5, a layer of insulation 6 and an abrasion-resistant coating 7. In the corresponding manner, the second conductor 4 comprises a conductor wire 8, a layer of insulation 9 and an abrasion-resistant coating 10. The function of the abrasion-resistant coating 7, 10 is to prevent the polymer coating on the first conductor 3 and second conductor 4 from wearing off. The polymer coatings are about 10 µm thick, while the abrasion-resistant coatings 7, 10 are one or a few microns thick and made from titanium nitride. In the prior art, the thickness of the polymer coating exceeds 60 µm. The 50 µm reduction may seem modest, but if it is assumed that the conductors 3, 4 are coiled with a specific internal diameter, the lead 1 will have an external diameter of about 0.7 mm. The reduction in the thickness of the polymer coating means that the diameter of the lead 1 can be reduced by about 200 µm, i.e. 0.2 mm representing a reduction by more than 25%.

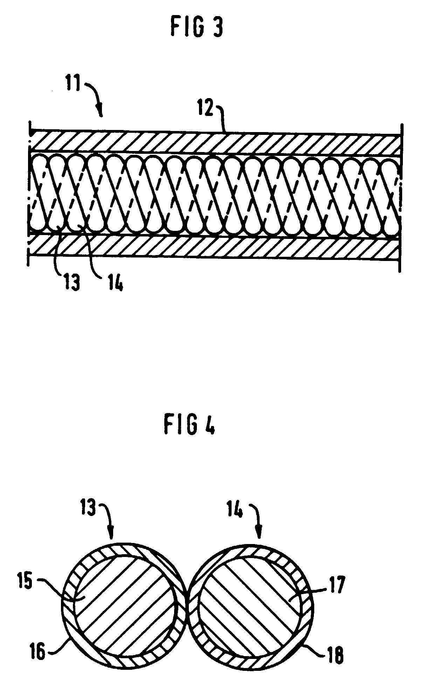

In FIG. 3 is shown an electrode lead 11 consisting of an insulating sleeve 12, a first conductor 13 and a second conductor 14. The figure only illustrates a middle section of the electrode lead 11, which has a connector contact at one end for coupling the electrode lead 11 to a stimulation pulse generator (not shown) such as a pacemaker, and at least one stimulation electrode at its other end for delivering stimulation pulses from the stimulation pulse generator to surrounding tissue, such as a heart.

The first conductor 13 and the second conductor 14 have the cross-section shown in FIG. 4. The first conductor 13 comprises a conductor wire 15 with an electrically insulating and abrasion-resistant coating 16. In the corresponding manner, the second conductor 14 is composed of a conductor wire 17 and an electrically insulating and abrasion-resistant coating 18. The purpose of the electrically insulating and abrasion-resistant coatings 16, 18 is to insulate the conductor wires 15, 17 from one another and minimize the risk of any short-circuit occurring because the layers wear off when the conductors 13, 14 rub against each other. The electrically insulating and abrasion-resistant layers 16, 18 are only a few micrometers thick and made of D.L.C. (diamond-like carbon).

## Claims

1. A multipolar electrode lead (1; 11) for a medical apparatus devised to deliver stimulation pulses to living tissue, said electrode lead (1; 11) comprising an insulating sheath (2; 12) in which at least a first conductor (3; 13) and a second conductor (4; 14) are coiled, at least the first conductor (3; 13) being covered with a coating (6; 16) which comprises either:
- a first electrically insulating layer (6) and a second, outer abrasion-resistant layer (7) which is not a polymer, or
- a single electrically insulating layer (16) which is also abrasion-resistant and is not a polymer.

2. An electrode lead of claim 1, wherein the at least first and second conductors (3; 13; 4; 14) are covered with the same electrically insulating and abrasion-resistant coating (6; 7; 9; 10; 16; 18).

3. An electrode lead of any of the claims 1 or 2, wherein the electrically insulating and abrasion-resistant coating (16, 18) is made from the material D.L.C. (diamond-like carbon).

4. An electrode lead of claim 3, wherein the electrically insulating and abrasion-resistant coating (16, 18) is applied in a layer with a thickness of 0.1 to 10 µm, preferably between 1 and 5 µm.

5. An electrode lead of claim 1 or 2, wherein the insulating coating (6, 7, 8, 10) comprises a first inner layer (6, 8) of an electrically insulating material and a second outer layer (7, 10) of an abrasion-resistant material.

6. An electrode lead of claim 5, wherein the electrically insulating material is a polymer.

7. An electrode lead of any of the claims 5 or 6, wherein the abrasion-resitant layer (7, 10) is made of any of the materials titanium nitride, aluminium oxide or DLC (diamond-like carbon).

8. An electrode lead of any of the claims 5 - 7, wherein the abrasion-resistant layer (7, 10) is deposited in a thickness of 0.1 to 10 µm, preferably between 1 and 5 µm.

## Patentansprüche

1. Eine mehrpolige Elektrodenleitung (1; 11) für ein medizinisches Gerät, ausgebildet, um Stimulationsimpulse an lebendes Gewebe abzugeben, welche Elektrodenleitung (1; 11) einen isolierenden Mantel (2; 12) aufweist, in dem zumindest ein erster Leiter (3; 13) und ein zweiter Leiter (4; 14) gewickelt sind, wobei zumindest der erste Leiter (3; 13) von einer Schicht (6; 16) bedeckt ist, die entweder:
- eine erste, elektrisch isolierende Lage (6) und eine zweite, äußere, abriebsresistente Lage (7), die kein Polymer ist, oder
- eine einzige, elektrisch isolierende Lage (16), die auch abriebsresistent und kein Polymer ist, aufweist.

2. Eine Elektrodenleitung nach Anspruch 1, bei der die zumindest erste und zweite Leiter (3; 13; 4; 14) von der selben elektrisch isolierenden und abriebsresistenten Schicht (6,7;9,10;16;18) bedeckt sind.

3. Eine Elektrodenleitung nach einem der Ansprüche 1 oder 2, bei der die elektrisch isolierende und abriebsresistente Schicht (16, 18) aus dem Material D.L.C. ( diamond-like carbon) hergestellt ist.

4. Eine Elektrodenleitung nach Anspruch 3, bei der die elektrisch isolierende und abriebsresistente Schicht (16, 18) in einer Lage mit einer Dicke von 0.1 bis 10 µm, vorzugsweise zwischen 1 und 5 µm, aufgetragen ist.

5. Eine Elektrodenleitung nach Anspruch 1 oder 2, bei der die isolierende Schicht (6, 7, 8, 10) eine erste innere Lage (6, 8) aus einem elektrisch isolierendem Material und eine zweite äußere Lage (7, 10) aus einem abriebsresistenten Material aufweist.

6. Eine Elektrodenleitung nach Anspruch 5, bei der das elektrisch isolierende Material ein Polymer ist.

7. Eine Elektrodenleitung nach einem der Ansprüche 5 oder 6, bei der die abriebsresistente Lage (7, 10) aus einem der Materialien Titannitrid, Aluminiumoxid oder DLC (diamond-like carbon) hergestellt ist.

8. Eine Elektrodenleitung nach einem der Ansprüche 5 - 7, bei der die abriebsresistente Lage (7, 10) in einer Dicke von 0.1 bis 10 µm, vorzugsweise zwischen 1 und 5 µm, aufgetragen ist.

## Revendications

1. Câble (1; 11) d'électrode multipolaire pour un dispositif médical conçu pour délivrer des impulsions de stimulation à du tissu vivant, le câble (1;11) d'électrode comportant une gaine (2;12) isolante, dans laquelle au moins un premier conducteur (3;13) et un second conducteur (4;14) sont enroulés, au moins le premier conducteur (3;13) étant recouvert d'un revêtement (6; 16) qui comprend soit:
- une première couche (6) isolante électriquement et une seconde couche (7) extérieure résistante à l'abrasion qui n'est pas un polymère, soit
- une seule couche (16) isolante électriquement, qui est également résistante à l'abrasion et qui n'est pas un polymère.

2. Câble d'électrode suivant la revendication 1, dans lequel lesdits au moins un premier et second conducteurs (3:13;4;14) sont recouverts du même revêtement (6,7;9,10;16;18) résistant à l'abrasion et isolant électriquement.

3. Câble d'électrode suivant l'une quelconque des revendications 1 ou 2, dans lequel le revêtement (16,18) résistant à l'abrasion et isolant électriquement est réalisé à partir du matériau D.L.C. (carbone analogue à du diamant).

4. Câble d'électrode suivant la revendication 3, dans lequel le revêtement (16,18) résistant à l'abrasion et isolant électriquement est appliqué en une couche d'une épaisseur de 0,1 à 10 µm, de préférence entre 1 et 5 µm.

5. Câble d'électrode suivant la revendication 1 ou 2, dans lequel le revêtement (6,7,8,10) isolant comprend une première couche (6,8) intérieure en un matériau isolant électriquement et une seconde couche (7,10) extérieure en un matériau résistant à l'abrasion.

6. Câble d'électrode suivant la revendication 5, dans lequel le matériau isolant électriquement est un polymère.

7. Câble d'électrode suivant l'une quelconque des revendications 5 ou 6, dans lequel la couche (7,10) résistant à l'abrasion est en un matériau quelconque parmi les matériaux que sont le nitrure de titane, l'oxyde d'aluminium ou le DLC (carbone analogue à du diamant).

8. Câble d'électrode suivant l'une quelconque des revendications 5 à 7, dans lequel la couche (7,10) résistant à l'abrasion est déposée en une épaisseur de 0,1 à 10 µm, de préférence de 1 et 5 µm.
